# FASCICULE DE BREVET EUROPEEN

(11) **EP 1 269 990 B1**
(45) Date de publication et mention de la délivrance du brevet: **13.10.2010**
(21) Numéro de dépôt: 02291599.5
(22) Date de dépôt: 26.06.2002
(51) Int. Cl.: A61K 8/06, A61Q 19/08

(54) **Composition cosmétique ou dermatologique comprenant des dérivés d'acylaminoamide**
Acylaminoamid-Derivate enthaltende kosmetische oder dermatologische Zusammensetzung
Cosmetic or dermatological composition comprising acylaminoamide derivatives

(30) Priorité: 26.06.2001 FR 0108436
(43) Date de publication de la demande: 02.01.2003
(73) Titulaire: L'Oréal, 75008 Paris (FR)
(72) Inventeur: Breton, Lionel, 78000 Versailles (FR)
(74) Mandataire: Catherine, Alain

(56) Documents cités:
- EP-A- 0 704 210
- EP-A- 1 053 745
- EP-A- 1 088 548
- WO-A-00/12467
- DE-A- 2 322 232
- FR-A- 2 810 033
- US-A- 5 234 909
- NAKAMURA M ET AL: "A two-step, one-pot synthesis of diverse N-pyruvoyl amino acid derivatives using the Ugi reaction" BIOORGANIC & MEDICINAL CHEMISTRY LETTERS, OXFORD, GB, vol. 10, no. 24, 18 décembre 2000 (2000-12-18), pages 2807-2810, XP004225356 ISSN: 0960-894X

## Description

La présente invention se rapporte au domaine des compositions cosmétiques ou dermatologiques. Elle est relative à de nouvelles compositions cosmétiques ou dermatologiques comprenant une association entre un composé inhibiteur de l'élastase de la famille des N-Acylaminoamides de formule définie dans la revendication 1 et au moins un agent myorelaxant. Cette composition est préférentiellement destinée à améliorer les signes cutanés du vieillissement et/ou du photovieillissement, et notamment les rides, en ralentissant les altérations du tissu conjonctif et en améliorant l'état fonctionnel de la peau tout en apportant une action myorelaxante.

La peau humaine est constituée de deux compartiments à savoir un compartiment superficiel, l'épiderme, et un compartiment profond, le derme.
L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses.

Par ailleurs, il est connu que les muscles peauciers du visage sont sous le contrôle des afférences nerveuses motrices du nerf facial. D'autre part, une sous-population de fibroblastes du derme (que l'on appelle myofibroblastes) présente certaines caractéristiques communes avec le tissu musculaire. Dans certaines situations pathologiques et thérapeutiques, le contrôle nerveux de l'ensemble du tissu musculaire de la face joue un rôle prépondérant sur l'apparition des rides du visage. Ainsi, dans les atteintes du nerf facial, dans lesquelles la transmission de l'influx nerveux est interrompue et/ou amoindrie, on assiste dans le territoire d'innervation à une paralysie des muscles du visage. Cette paralysie faciale se traduit, entre autres signes cliniques, par une atténuation, voire une disparition des rides. Par ailleurs, il a été montré que la toxine botulique, utilisée à l'origine pour traiter les spasmes, pouvait agir sur les états de spasticité musculaire (voir A. Blitzer et al., Arch. Otolaryngol. Head Neck Surg., 1993, 199, pages 1018 à 1022) et sur les rides de la glabelle qui sont les rides inter sourcilières (voir J.D. Carruters et al., J. Dermatol. Surg. Oncol., 1992, 18 pages 17 à 21). En conséquence, il est donc possible d'agir par une action pharmacologique sur la composante neuromusculaire des rides.

Dans le système nerveux périphérique, la jonction entre un nerf et un muscle strié constitue la plaque neuro-musculaire, en amont de laquelle se trouve la voie nerveuse afférente appelée motoneurone. Par ailleurs, les membranes cellulaires de chaque fibre nerveuse mais également des cellules musculaires comportent de nombreux canaux ioniques, et notamment des canaux calciques, ou des canaux chlore, aptes à laisser traverser de façon contrôlée respectivement le Ca⁺⁺ ou le Cl⁻.

Dans les cellules musculaires, le messager final de la contraction musculaire est également le calcium dont l'élévation dans le cytoplasme du myocyte permet l'activation de la machinerie contractile. Il est généralement admis que pendant la phase de contraction, les filaments minces d'actine glissent entre les filaments épais de myosine entraînant ainsi le raccourcissement des sarcomères et par voie de conséquence un mouvement contractile de la cellule et globalement de la fibre.

A l'état relaché, l'actine n'est pas accessible aux ponts de myosine parce qu'elle est associée à un autre complexe protéique constitué par la troponine et la tropomyosine.

Quand le calcium se lie au complexe troposine-tropomyosine, les molécules d'actine deviennent accessibles et le phénomène contractile peut alors débuter.

Elle consiste en une réaction ATPasique qui produit l'énergie pour le recyclage des ponts.

Le rôle du Ca²⁺ au niveau des protéines contractiles des muscles striés est un rôle activateur (desinhibiteur) de l'activité ATPasique.

Le relâchement du muscle strié a lieu quand les tubules transverses et la membrane cellulaire sont repolarisés, ceci permet le retour de la concentration intra-cytoplasmique cellulaire du Ca²⁺ à une valeur de 10⁻⁷ M, inférieure au seuil d'activation des enzymes intracellulaires telle que l'ATPase (seuil d'activation qui avoisine 1 à 2 logarithmes de concentration supérieure).

Le calcium n'est pas un activateur *per se* mais il le devient après formation d'un complexe avec la *calmoduline,* petite protéine (18.000 daltons). Le cycle contraction-relâchement est dû aux variations de la concentration du calcium cytoplasmique de 10⁻⁷ (inactif) à 10⁻⁵ M (actif).

La régulation de la concentration cytoplasmique intracellulaire du Ca²⁺ n'est possible que parce que les efflux cytoplasmiques de calcium corrigent les influx cytoplasmiques. Les échanges de Ca++ intra-cytoplasmiques se font, soit vis-à-vis des vésicules de réserve intracellulaire, soit vis-à-vis de l'extérieur de la cellule. Dans les deux cas, le Ca++ n'est pas disponible dans le cytoplasme. Ces échanges ne peuvent être assurés que par une expulsion du calcium cytoplasmique intracellulaire par un ou des mécanismes dits actifs, aptes à surmonter le gradient de potentiel électrochimique évoqué plus haut.

Deux types de mécanismes peuvent intervenir : une pompe à calcium qui expulse activement les cations aux dépens de l'hydrolyse d'ATP et un mouvement de Ca²⁺ couplé à un mouvement de Na⁺. Dans la plupart des cellules, la pompe à calcium ATP-dépendante opère plus efficacement en présence de la *calmoduline* qui augmente son affinité.

Afin de mieux décrire les changements de perméabilité au calcium, il est actuellement habituel, de considérer que cette perméabilité correspond à l'ouverture de canaux calcium membranaires, canaux opérés par des variations du potentiel de la membrane (VOC) ou l'activation des récepteurs membranaires (ROC). A ce jour, trois types VOC de canaux calcium (L, N, T) ont été identifiés. D'autre part, il est vraisemblable que ces canaux calciques présentent des spécificités tissulaires.

En 1965, T. Godfraind a mis en évidence que la perméabilité de la membrane au calcium pourrait être inhibée par des agents pharmacologiques, ce qui constituerait le mécanisme commun sur lequel agiraient des antagonistes du Ca⁺⁺.

Indépendamment dès agents bloqueurs du Ca++, les agents ouvreurs de canaux chlore (Cl-), tels que ceux décrits dans la demande de brevet EP 0704210 constituent également des myorelaxants utilisables selon l'invention. Les composés ouvreurs de canaux chlore constituent des « agonistes de canaux chlore » au sens de l'invention.

On comprend donc de ce qui précède que la contraction ou l'hypercontraction de certains muscles de la face, ou de certaines cellules contractiles du derme comme les myofibroblates, résulte en l'apparition de rides.

Cette activation musculaire est elle-même induite par différents mécanismes faisant notamment intervenir des échanges ioniques Cl-, Ca++ et du calcium intracellulaire.

La demanderesse a maintenant découvert après de nombreux tests cliniques, que la fibre musculaire contractile, qui se trouve sous le contrôle direct de l'influx neuro-moteur, joue un rôle essentiel dans la formation des rides et que la modulation de l'influx neuro-moteur et le contrôle de la contraction des fibres musculaires, jouent un rôle essentiel dans la formation des rides. Ainsi la modulation de la contraction motrice atténue non seulement les rides mais également les ridules et présente aussi un effet de " lissage " sur le microrelief cutané. Elle a aussi trouvé que les tissus cutané et sous-cutané comportent des canaux calcium, ce qui, jusqu'à présent, n'a pas été envisagé.

Par ailleurs, à long terme, la somme des micro-stress cutanés, par exemple générés par le vieillissement ou par une exposition prolongée aux UV (dans ce cas, il s'agit de photovieillissement), peut entraîner une perte plus ou moins accélérée de l'élasticité naturelle de la peau. Le réseau formé par les fibres élastiques du tissu conjonctif sous-jacent et des espaces extracellulaires peut alors progressivement être destructuré. Il s'en suit un vieillissement accéléré de la peau (peau ridée et/ou moins souple) via l'altération du réseau élastique dermique, ainsi qu'une accentuation des rides (rides plus profondes).

Lors d'un stress, les kératinocytes libèrent des médiateurs biologiques (appelés facteurs chimioattractants) qui possèdent la capacité d'attirer certaines cellules inflammatoires du compartiment sanguin vers le tissu cutané . Ces cellules sont responsables de la genèse puis de l'entretien d'une irritation locale pendante de l'état de vieillissement.

Parmi les facteurs chimio-attractants pouvant être produits par les kératinocytes « stressés », l'interleukine 8 (IL-8) est plus spécifiquement responsables du recrutement des polynucléaires neutrophiles. Ces cellules infiltrant les zones irritées ou agressées libèrent alors des enzymes parmi lesquelles l' élastase leucocytaire et d'autres protéases (metalloprotéinases, serines protéases etc...)

Sous l'action de l'élastase, les fibres élastiques de soutien extracellulaire du tissu conjonctif sont dégradées. En synergie avec la cathepsine G, l'élastase leucocytaire peut même dissocier l'intégrité de l'épiderme en élargissant les espaces intercellulaires inter-kératinocytaires (Ludolph-Hauser et al Exp. Dermatol. 1999 8(1) 46-52). L'élastase leucocytaire a récemment été incriminée dans l'entretien des escarres et la survenue des ulcères veineux des jambes, via son activité de dégradation de la fibronectine (Herrick S et al Lab.Invest. 1997(3) 281-288. La somme des micro-stress localisés de dégradation (suite par exemple à une exposition prolongée au soleil) peut aboutir au long terme à une perte accélérée de l'élasticité naturelle de la peau. Le réseau des fibres élastiques du tissu conjonctif sous jacent et des espaces extracellulaires est alors progressivement déstructuré. Cette dégradation accélérée peut se cumuler avec le processus de vieillissement normal de la peau qui se caractérise par une plus grande sensibilité des fibres élastiques à l'action de l'élastase (Stadler R & Orfanos CE Arch. Dermatol. Res. 1978 262 (1) 97-111.

Il est connu dans l'état de la technique d'apporter, dans le tissu cutané, des molécules capables de ralentir l'activité de dégradation des fibres élastiques des espaces intercellulaires.

US 5 234 909 décrit des compositions cosmétiques comprenant une N-acylaminoamide dont l'action soignante et hydratante est établie, et en particulier l'action contre les rides.

La présente invention a pour but de proposer une solution à ces différents problèmes, et notamment de proposer de nouvelles compositions susceptibles d'être utilisées en cosmétique ou en pharmaceutique pour limiter le vieillissement de la peau, qu'il soit chronobiologique ou photo-induit, et notamment le vieillissement généré par une diminution de l'élasticité de la peau et/ou par une dégradation du collagène dans la structure des tissus.

La solution technique selon l'invention consiste à apporter, en plus de l'élément régulateur en aval de l'activité élastasique (i.e le dérivé N-Acylaminoamide, l'inhibiteur de l'activité enzymatique de l' élastase leucocytaire) un ou plusieurs actifs capables de réguler aussi la composante myofibrillaire cutanée.

En conséquence, l'invention a pour objet une composition cosmétique ou dermatologique caractérisée en ce qu'elle comprend une association entre un composé inhibiteur de l'élastase de la famille des N-Acylaminoamides de formule définie dans la revendication 1 et au moins un agent myorelaxant.

Préférentiellement, l'agent myorelaxant est choisi parmi un inhibiteur des canaux calciques et/ou un agoniste des canaux chlore.

Un autre objet de l'invention consiste en un procédé de traitement cosmétique de la peau du corps ou du visage, y compris le cuir chevelu, dans lequel on applique sur la peau une composition cosmétique telle que définie ci-dessus.

Il a en effet été constaté que les composés de formule (I) présentaient une activité inhibitrice de l'activité des élastases, et qu'ils pouvaient donc être employés pour limiter et/ou combattre la dégradation des fibres élastiques.

Il s'en suit qu'ils peuvent être employés dans ou pour la préparation d'une composition, les composés ou la composition étant destinés à traiter, de manière préventive et/ou curative, les signes cutanés du vieillissement.

L'association nouvelle des composés N-Acylaminoamides de formule définie dans la revendication 1 avec au moins un agent myorelaxant permet de renforcer significativement l'effet antivieillissement du tissu matriciel, par l'apport d'un effet de relâchement du tissu cutané et/ou du tissu sous-cutané.

Ainsi l'utilisation conjointe dans une composition cosmétique d'un inhibiteur de l'élastase de la famille des N-Acylaminoamides de formule définie dans la revendication 1 et d'un agent myorelaxant, préférentiellement du type antagoniste Ca⁺⁺ et/ou ouvreurs de canal Cl^{-,} concourt à une plus grande activité antirides via une action protectrice et régulatrice sur le tissu de soutien de la peau et via une activité myorelaxante sur toutes les myofibrilles du derme et/ou de l'hypoderme.

Selon l'invention, l'élément régulateur de l'activité élastasique est combiné à un ou plusieurs actifs capables de réguler la composante myofibrillaire cutanée.

La composition obtenue est destinée à traiter les désordres du vieillissement et/ou à être plus spécifiquement destinée à traiter tous les désordres cutanés associés à une trop grande prolifération des bactéries et/ou des levures (P. Ovale, P. Acnes, A. Aureus) cutanées.

Préférentiellement, cette nouvelle association est utilisée dans des préparations cosmétiques de soin et/ou d'hygiène des zones exposées au soleil (scalp, corps, visage, lèvres), dans les préparation cosmétiques de soin et/ou d'hygiène des zones ulcérées, dans les dentifrices ou lotions de rinçage buccales et, de façon générale, dans toutes les préparations cosmétiques dites " anti-vieillissement cutané " qui ont pour objectif de ralentir la destructuration chronobiologique des tissus de soutien et de l'architecture des éléments matriciels cutanés. Plus particulièrement, cette association sera réservée aux peaux comédogènes et ou acnéiques, ainsi qu'aux peaux ayant subit des atteintes modérées comme des éraflures, griffures, brûlures du premier degré ou brûlures de degrés supérieurs mais en voie de resorption etc

Sans vouloir être lié par une quelconque théorie, le demandeur considère que le fait d'apporter, au niveau des kératinocytes des couches superficielles de la peau, des composés susceptibles de ralentir l'activité de dégradation des fibres élastiques des espaces intercellulaires, peut permettre de diminuer ce phénomène de vieillissement accéléré de la peau, dû à des stress cutanés superficiels et que l'association de ces composés avec un inhibiteur de canaux calciques ou un antagoniste de canaux chlore renforce considérablement leurs effets.

### Composés N-acylamino-amides préférés

Les composés susceptibles d'être employés dans la présente invention répondent donc à la formule (I) suivante : 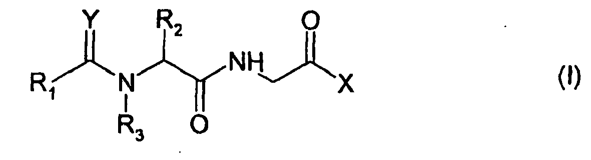 dans laquelle :
- le radical Y représente O ou S,
- le radical R1 représente :
   - (i) un atome d'hydrogène;
   - (ii) un radical hydrocarboné, linéaire, ramifié ou cyclique, saturé ou insaturé, ayant 1 à 18 atomes de carbone,
      éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR; -P(O)-(OR)₂; -SO₂-OR;
      avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR";-COR"; avec R" représentant un radical hydrocarboné, linéaire, ramifié ou cyclique, saturé ou insaturé, ayant 1 à 6 atomes de carbone, éventuellement halogéné, voire perhalogéné;
   - (iii) un radical choisi parmi les radicaux -OR; -NH₂; -NHR; -NRR'; -NH-COR; -COOR; -COR ;
      avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; - SR"; -S-COR"; -NH₂; -NHR"; -NH-COR"; -Hal (halogène); -CN; -COOR";-COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R2 représente un radical hydrocarboné, linéaire, ramifié ou cyclique, saturé ou insaturé, ayant 1 à 18 atomes de carbone,
   éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR;
   avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
   lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH;
- SR"; -S-COR"; -NH₂; -NHR"; -NH-COR"; -Hal (halogène); -CN; -COOR";-COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R3 représente un radical choisi parmi ceux de formule (II) ou (III) :

   (II) -A-C₆H_{(5-y)}-B_{y}

   (III) -C₆H_{(5-y')}B_{y'}
dans lesquelles :
- y est un entier compris entre 0 et 5 inclus, et y' est un entier compris entre 1 et 5 inclus;
- A est un radical divalent hydrocarboné, linéaire ou ramifié, saturé ou insaturé, ayant 1 à 18 atomes de carbone,
   éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
   lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR";-COR"; avec R" représentant un radical hydrocarboné, linéaire, ramifié ou cyclique, saturé ou insaturé, ayant 1 à 6 atomes de carbone, éventuellement halogéné, voire perhalogéné;
- B est un radical hydrocarboné, linéaire ou ramifié, saturé ou insaturé, ayant 1 à 18 atomes de carbone,
   éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
   lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR";-COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical X représente un radical choisi parmi -OH, -OR₄, -NH₂, -NHR₄,-NR₄R₅, -SR₄, -COOR₄; -COR₄;
   avec R₄ et R₅ représentant, indépendamment l'un de l'autre, un radical hydrocarboné, linéaire, cyclique ou ramifié, saturé ou insaturé, ayant 1 à 6 atomes de carbone, éventuellement substitué par 1 à 5 groupements, identiques ou différents, choisis parmi -OH; -OR; -O-COR; -SH; -SR; -S-COR;-NH₂; -NHR; -NRR'; -NH-COR; -Hal (halogène, voire perhalogène); -CN;-COOR; -COR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné; lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
   lesdits radicaux R₄ et R₅ pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR";-COR"; avec R" représentant un radical hydrocarboné, linéaire, ramifié ou cyclique, saturé ou insaturé, ayant 1 à 6 atomes de carbone, éventuellement halogéné, voire perhalogéné.

Sont également compris dans cette définition, les sels d'acide minéral ou organique desdits composés, ainsi que leurs isomères optiques, sous forme isolée ou en mélange racémique.

Par radical hydrocarboné, linéaire, cyclique ou ramifié, on entend notamment les radicaux de type alkyle, aryle, aralkyle, alkylaryle, alcényle, alcynyle.

Le groupement C₆H₅ présent dans le radical R3 doit être compris comme un groupement cyclique aromatique.

De préférence, le radical Y représente l'oxygène.

De préférence, le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué. Notamment, les substituants peuvent être choisis parmi -OH, -OR et/ou -P(O)-(OR)₂ avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R1 représente un radical méthyle, éthyle, propyle ou isopropyle, éventuellement substitué par un groupement -OH ou-P(O)-(OR)₂ avec R représentant méthyle, éthyle, propyle ou isopropyle.

De préférence, le radical R2 représente un radical hydrocarboné, linéaire, ramifié ou cyclique, saturé ou insaturé, ayant 1 à 12, notamment 1, 2, 3, 4, 5 ou 6, atomes de carbone, éventuellement substitué.

Notamment, les substituants peuvent être choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R2 représente un radical méthyle, éthyle, propyle, isopropyle, n-butyle, ter-butyle ou isobutyle.

De préférence, le radical R3 représente un radical de formule -C₆H_{(5-y')}-B_{y'} pour lequel y' = 1, 2 ou 3; ou un radical de formule -A-C₆H_{(5-y)}-B_{y} pour lequel y = 0, 1 ou 2.

De préférence, A est un radical divalent hydrocarboné, linéaire ou ramifié, saturé ou insaturé, ayant 1 à 12 atomes de carbone, éventuellement substitué.

Les substituants de A sont de préférence choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné.

De préférence, B est un radical hydrocarboné, linéaire ou ramifié, saturé ou insaturé, ayant 1 à 12 atomes de carbone, éventuellement substitué.

Les substituants de B sont de préférence choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R3 représente un groupement choisi parmi l'une des formules suivantes : 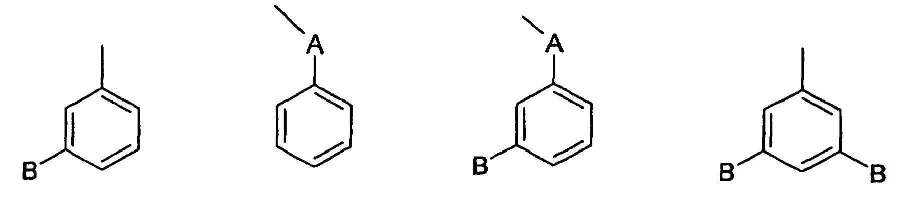 dans lesquelles A et B ont les significations ci-dessus.

Notamment, le radical divalent A peut être un méthylène, un éthylène, un propylène.

Le radical B est de préférence un radical méthyle, éthyle, propyle ou isopropyle, substitué par un ou plusieurs halogènes, notamment chlore, brome, iode ou fluor, et préférentiellement totalement halogéné (perhatogéné), tel que perfluoré. On peut en particulier citer le radical perfluorométhyle (-CF3) comme tout particulièrement préféré.

De préférence, le radical X représente un radical choisi parmi -OH ou-OR₄ avec R₄ représentant un radical hydrocarboné, linéaire, cyclique ou ramifié, saturé ou insaturé, ayant 1 à 6 atomes de carbone, éventuellement substitué.

Les substituants peuvent être choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical X représente un radical choisi parmi -OH,-OCH₃, -OC₂H₅, -O-C₃H₇ ou -OC₄H₉.

Parmi les composés particulièrement préférés, on peut citer :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-(benzyl-[(diethoxy-phosphoryl)-acétyl]-amino}-3-méthyl-butyrylamino) acétate d'éthyle.

Les composés selon l'invention peuvent être aisément préparés par l'homme du métier sur base de ses connaissances générales. On peut notamment faire réagir ensemble un acide carboxylique, un aldéhyde, un composé aminé et un isonitrile, selon la réaction de Ugi.

Bien entendu, lors de la synthèse des composés selon l'invention, et en fonction de la nature des différents radicaux présents sur les composés de départ, l'homme du métier pourra veiller à protéger certains substituants pour que ceux-ci n'interviennent pas dans la suite des réactions.

La quantité de composé à utiliser dans les compositions selon l'invention peut être aisément déterminée par l'homme du métier, en fonction de la nature du composé utilisé, de la personne à traiter et/ou de l'effet recherché. D'une manière générale, cette quantité peut être comprise entre 0,00001 et 20% en poids par rapport au poids total de la composition, notamment entre 0,001 et 10% en poids, et de préférence entre 0,05 et 5% en poids.

Les composés de formule (I) peuvent notamment être employés dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

### Agents myorelaxants préférés selon l'invention

### Inhibiteurs des canaux calciques

Pour qu'une substance soit reconnue comme un inhibiteur des canaux calcium, autrement appelé dans le texte inhibiteur calcique, elle doit pouvoir diminuer la concentration intracellulaire en calcium ou diminuer la liaison du calcium aux protéines intracellulaires comme par exemple la calmoduline, tel que cela est notamment décrit par exemple, par Galizzi, J.P et al, J. Biol. Chem. 1987, 262 p 6947 ou Y. Okamiya et al, Eur. J. Pharmacol. 1991, 205, p 49 ou J.A. Wagner et al, J. Neurosci. 1988, 8, p 3354 ou H.R Lee et al, Life Sci. 1984, 35 p 721 ou Schoemaker H. et Lauger S. Eur. J. Pharmacol. 1985, 111 p 273 ou encore I.J. Reynolds et al, J. Pharmacol. Exp. Ther. 1986, 237 p 731.

Une substance est reconnue comme relaxante au sens de l'invention lorsqu'elle montre un effet de relaxation sur un tissu musculaire contracté et/ou montrer un effet inhibiteur dans un modèle expérimental de jonction nerf-muscle (plaque motrice) notamment dans le modèle décrit par W. Steinbrecher dans : Electrodes for stimulation and bioelectric potentiel recording, Ed. Biomerstechnich, 1988, pages 96-98.

La quantité efficace d'inhibiteur d'au moins un canal calcique utilisable selon l'invention est bien entendu fonction de l'effet recherché et peut donc varier dans une large mesure.

Pour donner un ordre de grandeur, il est possible d'utiliser selon l'invention un inhibiteur d'au moins un canal calcique en une quantité représentant de 0,0001% à 10% du poids total de la composition et préférentiellement en une quantité représentant de 0,001% à 5% du poids total de la composition.

Parmi les antagonistes calciques, il faut considérer deux classes d'actifs, respectivement les agents actifs sur la membrane plasmique et les agents actifs à l'intérieur de la cellule.
**1) Agents actifs sur la membrane plasmique,** inhibiteurs de l'entrée du calcium :
   Les inhibiteurs de canaux calcique utilisables selon l'invention, sont les agents actifs sur la membrane plasmique, complexant le calcium et/ou inhibiteurs de l'entrée du calcium comme les phenylalkylamines comme par exemple le vérapamil, l'anipamil, le gallopamil, le dévapamil, le falipamil, le tiapamil, des dihydropyridines comme par exemple la nifédipine, l'amlodipine, la dazodipine, la félodipine, l'isradipine, la nicardipine, la nimodipine, la nisoldipine, la nitrendipine, la ryosidine, des benzothiazépines comme par exemple le Diltiazem, des diphenylpipérazines comme par exemple la cinnarizine, la flunarizine, l'alvérine et/ou ses sels organiques ou inorganiques et/ou ses dérivés chimiques, le manganèses et ses sels organiques et/ou inorganiques.
**2) Agents actifs à l'intérieur de la cellule (libération des réserves intracellulaires du Ca²⁺ ou alors inhibition de la formation du complexe Ca²⁺ Calmoduline)**
   Les agents actifs à l'intérieur de la cellule préférés selon l'invention sont ceux intervenant sur la libération des réserves intracellulaires du calcium ou alors sur l'inhibition de la formation du complexe calcium/calmoduline. Ce sont par exemple des agents intervenant au niveau du réticulum sarcoplasmique comme par exemple le dantrolène et le TMB-8, des antagonistes de la calmoduline comme par exemple la phénothiazine, la trifluopérazine, la chlorpromazine ou des dérivés du naphtalène ou des anesthésiques locaux comme la dibucaïne ou des antagonistes de la dopamine comme la pimozide, l'halopéridol ou le calmidazolium.
   Préférentiellement selon l'invention on utilise des agents actifs sur la membrane plasmique, inhibiteurs de l'entrée du calcium ou encore complexant le calcium. Très préférentiellement selon l'invention on utilise les inhibiteurs de l'entrée du calcium comme l'Alvérine et/ou ses sels et/ou ses analogues et/ou ses dérivés.

### Agonistes des canaux chlore

Il - Les agents ouvreurs de canaux Cl⁻ tels que décrits dans la demande EP 0704210 sont également revendiqués comme agents myorelaxants selon l'invention.

Il s'agit notamment des benzodiazepines, des analogues du GABA, mimétique du récepteur GABA ergique, mais également de la glycine et/ou de ses dérivés et de toutes autres substances qui concourt à l'ouverture du canal Cl⁻ de la cellule contractile et donc à une augmentation de l'excitabilité cellulaire par une augmentation significative de la différence de potentiel membranaire (ddp).

Une composition selon l'invention peut en outre être caractérisée en ce qu'elle comprend à la fois au moins un inhibiteur de canaux calciques et au moins un agoniste des canaux chlore.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.

Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Ladite phase aqueuse peut comprendre en outre des alcools tels que des monoalcools en C₁-C₆ et/ou des polyols tels que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale; minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux; un shampooing antiparasitaire;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

Les compositions selon l'invention trouvent une application préférée comme composition de soin de la peau du visage, de type anti-rides ou anti-age, et comme composition de protection solaire ou après-soleil.

La présente invention a également pour objet un procédé de traitement cosmétique de la peau du corps ou du visage, y compris le cuir chevelu, dans lequel on applique sur la peau une composition cosmétique comprenant une association entre un composé de la famille des N-acylamino-amides et au moins un inhibiteur de canaux calciques ou au moins un agoniste de canaux chlore, à laisser celle-ci en contact puis éventuellement à rincer.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs; application d'une lotion pour cuir chevelu sur cheveux mouillés; application de dentifrice sur les gencives.

L'invention a aussi pour objet un procédé de traitement cosmétique des rides et/ou des ridules, consistant à appliquer sur la peau une composition cosmétique comprenant dans un milieu cosmétiquement acceptable, une quantité efficace d'au moins un inhibiteur d'au moins un canal calcique.

Par milieu cosmétiquement acceptable, on entend compatible avec la peau, le cuir chevelu et/ou les muqueuses.

Le procédé de traitement cosmétique de l'invention peut être mis en oeuvre notamment en appliquant la composition cosmétique telle que définie ci-dessus, selon la technique d'utilisation habituelle de ces compositions. Par exemple : application de crèmes, de gels, de sérums, de lotions, de laits de démaquillage ou de compositions anti-solaires sur la peau ou de compositions pour spray.
L'invention est illustrée plus en détail dans les exemples suivants.

### Exemple 1

### Préparation du {2-[acétyl-(3-trifluorométhyl-phényl)-amino]-3-méthyl-butyrylamino} acétate d'éthyle de formule :

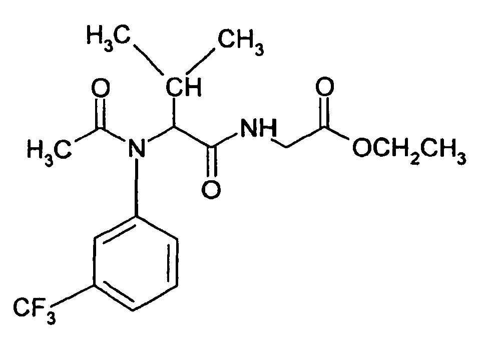

On mélange 0,63 ml d'isobutyraldéhyde et 1 ml de trifluorométhylamine (1,15 eq) dans 15 ml de méthanol, sous agitation. On laisse réagir 15 minutes à 20°C, puis on ajoute 0,46 ml d'acide acétique (1,15 eq) et on laisse réagir 10 minutes à 20°C. On ajoute alors 0,8 ml d'isocyanoacétate d'éthyle à 95% (1 eq) et on laisse réagir 48 heures à 20°C.

On concentre le milieu réactionnel au rotovapor et on purifie le résidu sur colonne de silice (éluant : heptane : 3 / acétate d'éthyle : 7; Rf = 0,5).
On obtient 2,45 g de composé sous forme de solide cireux, d'où un rendement de 91%.
RMN ¹H (200 MHz; CDCl3) δ ppm : 0,9 (6H;q), 1,3 (3H;t), 1,8 (3H;s), 2,3 (1H;m), 4,0 (2H,q), 4,2 (2H;q), 4,4 (2H;d), 7,3 (1H;t), 7,5 (4H;m)

### Exemple 2

### Préparation de l'acide {2-[acétyl-(3-trifluorométhyl-phényl)-amino]-3-méthyl-butyrylamino} acétique de formule :

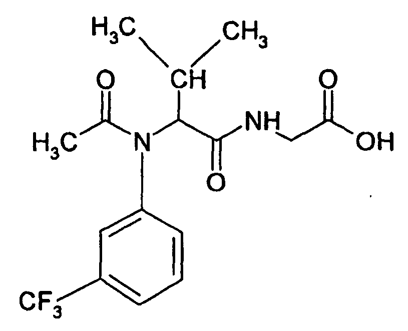

On solubilise 2 g de composé préparé à l'exemple 1 dans 30 ml d'acétone. On ajoute 30 ml de soude 2N et on laisse réagir pendant 6 heures à 20°C. On concentre le milieu réactionnel au rotovapor. On acidifie la phase aqueuse résiduelle à pH 2 par ajout d'HCI concentré puis on extrait par CH2Cl2.

La phase organique est concentrée à sec après séchage sur sulfate de sodium.

On obtient un résidu qui est solubilisé par un mélange eau basique à 10% d'éthanol, puis de nouveau acidifié par HCI concentré à pH 2. On extrait une nouvelle fois par Ch2Cl2 et on sèche la phase organique sur sulfate de sodium, on la filtre et on la concentre à sec sous vide au rotovapor.

On obtient 1,3 g de composé sous forme d'un solide légèrement marron clair, d'où un rendement de 70%.
RMN ¹H (200 MHz; DMSO) δ ppm : 0,9 (6H;q), 3,7 (2H;m), 1,8 (4H;m), 4,8 (2H;d), 7,6 (4H,m),8,4 (1H;t), 12,5 (1H;s)

### Exemple 3

On a déterminé in vitro l'activité anti-élastasique de composés selon l'invention, vis-à-vis de l'élastase leucocytaire humaine (ELH).

Le test est effectué de la manière suivante :

On laisse incuber, à 37°C, pendant 60 minutes, un substrat Me-OSAAPV-p-NA (Méthyl-O-succinate alanine alanine proline valine-p-nitroanilide) sur lequel est appliqué de l'ELH (40 milli-unités par ml) et 0,1% du composé à tester.

On détermine ensuite par spectrophotométrie le % d'inhibition de l'activité élastase témoin.

Les composés testés sont les suivants :
Composé A : acide {2-[acétyl-(3-trifluorométhyl-phényl)-amino]-3-méthyl-butyryl-amino} acétique
Composé B : (2-{benzyl-[(diethoxy-phosphoryl)-acétyl]-amino}-3-méthyl-butyryl-amino) acétate d'éthyle
Composé C : acide [2-(acétyl-benzyl-amino)-3-méthyl-butyrylamino] acétique
Composé D : [2-(acétyl-benzyl-amino)-3-méthyl-butyrylamino] acétate d'éthyle

On obtient les résultats suivants :

| Composé (concentration : 0,1%) | % d'inhibition de l'activité élastase témoin |
|---|---|
| Composé A | 67% |
| Composé B | 17% |
| Composé C | 20% |
| Composé D | 13% |

On détermine de la même manière le % d'inhibition de l'activité élastase témoin pour le composé A, à différentes concentrations.

On obtient les résultas suivants :

| Concentration du composé A | % d'inhibition de l'activité élastase témoin |
|---|---|
| 0,01% | 53% |
| 0,05% | 50% |
| 0,1% | 68% |
| 0,2% | 68% |

Le composé A provoque donc une forte inhibition de l'activité élastase, même en une quantité faible.

### Exemple 4

On a évalué l'activité ex vivo du composé de l'exemple 2 sur des peaux humaines en survie traitées par de l'élastase leucocytaire humaine (ELH).

Le test est effectué de la manière suivante :

Des coupes fraîches de peaux humaines, provenant de 2 donneurs différents, sont traitées pendant 2 heures, à 20°C, par 20 µl de solution tampon (pH 7,4) comprenant éventuellement 10 µg/ml d'ELH et éventuellement 0,1% du composé à tester, éventuellement préalablement mis en solution dans l'éthanol.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques | |
|---|---|---|
| | Peau 1 | Peau 2 |
| Témoin (peau non traitée) | 12,7% | 15,25% |
| Peau traitée avec ELH | 4,85% | 6,85% |
| Peau traitée avec ELH + composé de l'exemple 2 | 13,95% | 11,85% |

On constate donc que le composé selon l'invention génère une protection significative des peaux vis-à-vis de la destruction des fibres élastiques induite par l'élastase.

### Exemple 5

On a évalué l'activité ex vivo du composé de l'exemple 2 sur des peaux humaines en survie traitées par de l'élastase leucocytaire humaine (ELH).

Le test est effectué de la manière suivante :

Des fragments de peau humaine normale provenant de trois donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).

Le milieu de culture est renouvelé tous les trois jours.

On ajoute sur les fragments de peau éventuellement 0,5 µg d'ELH par mi de milieu de culture.

On ajoute également, tous les deux jours, 5 µl du composé à tester, préalablement mis en solution à 0,2% en poids dans l'éthanol.

Les peaux sont maintenues en survie pendant 10 jours à 37°C. Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques |
|---|---|
| Témoin (peau non traitée) | 7,4% |
| Peau traitée avec ELH | 5,1% |
| Peau traitée avec ELH + composé de l'exemple 2 | 7,1% |

On constate donc que le composé selon l'invention génère une protection significative des peaux vis-à-vis de la destruction des fibres élastiques induite par l'élastase.

### Exemple 6

On a évalué l'activité du composé de l'exemple 2 sur des peaux humaines en survie irradiées par des UVA (8 J/cm²).

Le test est effectué de la manière suivante :

Des fragments de peau humaine normale provenant de quatre donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).

Le milieu de culture est renouvelé tous les trois jours.

On ajoute sur les fragments de peau, tous les deux jours, 5 µl du composé à tester, préalablement mis en solution à 0,2% dans l'éthanol.

Les peaux sont maintenues en survie pendant 7 jours à 37°C.

Les peaux sont irradiées une seule fois à 8 J/cm² (lampe Vilbert-Lourmat RMX-3W).

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | Analyse morphométrique des fibres élastiques (derme superficiel) | Analyse morphométrique du collagène (derme superficiel) |
|---|---|---|
| Peau non traitée | 6,75% | 87% |
| Peau traitée par des UVA (8 J/cm²) | 3,9% | 81% |
| Peau traitée par des UVA (8 J/cm²) + composé | 6,8% | 92% |

On constate que le composé selon l'invention a bien une activité vis-à-vis de la dégradation des fibres élastiques dans le derme superficiel de peaux irradiées par des UVA.

Ce composé présente également un effet adéquat sur la protection du collagène.

### Exemple 7 : composition pour application topique

On prépare l'émulsion suivante de façon classique (% en poids) :
- composé de l'Exemple 2 1 %
- Diazepam 0,1 %
- isostéarate de propylène glycol 13 %
- polyéthylène glycol (8 OE) 5 %
- propylène glycol 3 %
- pentylène glycol 3 %
- stéarate de glycéryle et stéarate de polyéthylène glycol (100 OE) 5 %
- mono-stéarate de sorbitane oxyéthyléné (20 OE) 0,5 %
- alcool cétylique oxyéthyléné (20 OE) oxypropyléné (5 OP) 1 %
- gélifiants 0,5 %
- benzoates d'alkyle en C₁₂₋₁₅ 4 %
- éthanol 3 %
- hydroxyde de sodium 0,12 %
- conservateurs qs
- eau qsp 100 %

### Exemple 8 : crème de soin du visage

On prépare l'émulsion huile-dans-eau suivante de façon classique (% en poids) :
- Composé de l'exemple 2 %
- Alvérine 2 %
- Stearate de glycérol 2 %
- Polysorbate 60 (Tween 60® vendu par la société ICI) 1 %
- Acide stéarique 1,4 %
- Triéthanolamine 0,7 %
- Carbomer 0,4 %
- Fraction liquide du beurre de karité 12 %
- Perhydrosqualène 12 %
- Antioxydant qs
- Parfum qs
- Conservateur qs
- Eau qsp 100 %

### Exemple 9 : lait pour le visage

On prépare le lait suivant de façon classique (% en poids) :
- Huile de vaseline 7 %
- Composé de l'exemple 2 1 %
- Alvérine 2 %
- Gluconate de Manganèse 2 %
- Glycine (Glycocolle) 5 %
- Monostéarate de glycéryle, stéarate de polyéthylène glycol (100 OE) 3 %
- Polymère carboxyvinylique 0,4 %
- Alcool stéarylique 0,7 %
- Protéines de soja 3 %
- NaOH 0,4 %
- Conservateur qs
- Eau qsp 100 %

### Exemple 10 : lotion antichute

On prépare la lotion suivante de façon classique (% en poids) :
- composé de l'Exemple 2 1 %
- Alvérine 2 %
- propylène glycol 23 %
- éthanol 55 %
- Aminexil 1,5 %
- eau qsp 100 %

On peut appliquer cette lotion antichute sur le scalp des individus alopéciques.

## Revendications

1. Composition cosmétique ou dermatologique **caractérisée en ce qu'**elle comprend une association entre un composé inhibiteur de l'élastase de la famille des N-Acylaminoamides et au moins un agent myorelaxant, ledit composé inhibiteur de la famille des N-Acylaminoamides est un composé de formule (I) : 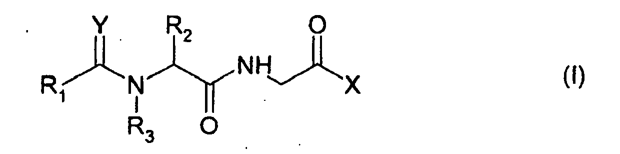 dans laquelle :
- le radical Y représente O ou S,
- le radical R1 représente :
- (i) un atome d'hydrogène;
- (ii) un radical hydrocarboné, linéaire, ramifié ou cyclique, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR; -P(O)-(OR)₂; -SO₂-OR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR";-COR"; avec R" représentant un radical hydrocarboné, linéaire, ramifié ou cyclique, saturé ou insaturé, ayant 1 à 6 atomes de carbone, éventuellement halogéné, voire perhalogéné;
- (iii) un radical choisi parmi les radicaux -OR; -NH₂; -NHR; -NRR'; -NH-COR; -COOR; -COR ;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné, lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; - COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R2 représente un radical hydrocarboné, linéaire, ramifié ou cyclique, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; - COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R3 représente un radical choisi parmi ceux de formule (II) ou (III) :
(II) -A-C₆H_{(5-y)}-B_{y}
(III) -C₆H_{(5-y')}-B_{y'}
dans lesquelles :
- y est un entier compris entre 0 et 5 inclus, et y' est un entier compris entre 1 et 5 inclus;
- A est un radical divalent hydrocarboné, linéaire ou ramifié, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; - COR"; avec R" représentant un radical hydrocarboné, linéaire, ramifié ou cyclique, saturé ou insaturé, ayant 1 à 6 atomes de carbone, éventuellement halogéné, voire perhalogéné;
- B est un radical hydrocarboné, linéaire ou ramifié, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; - COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical X représente un radical choisi parmi -OH, -OR₄, -NH₂, -NHR₄, - NR₄R₅, -SR₄, -COOR₄; -COR₄;
avec R₄ et R₅ représentant, indépendamment l'un de l'autre, un radical hydrocarboné, linéaire, cyclique ou ramifié, saturé ou insaturé, ayant 1 à 6 atomes de carbone, éventuellement substitué par 1 à 5 groupements, identiques ou différents, choisis parmi -OH; -OR; -O-COR; -SH; -SR; -S-COR; - NH₂; -NHR; -NRR'; -NH-COR; -Hal (halogène, voire perhalogène); -CN; - COOR; -COR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné; lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
lesdits radicaux R₄ et R₅ pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; - OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
ses sels d'acide minéral ou organique, ses isomères optiques, sous forme isolée ou en mélange racémique.

2. Composition selon la revendication 1 dans laquelle le composé de formule (I) est tel que :
- le radical Y représente l'oxygène, et/ou
- le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué, et/ou
- les substituants de R1 sont choisis parmi -OH, -OR et/ou -P(O)-(OR)₂ avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R2 représente un radical hydrocarboné, linéaire, ramifié ou cyclique, saturé ou insaturé, ayant 1 à 12, notamment 1, 2, 3, 4, 5 ou 6, atomes de carbone, éventuellement substitué; et/ou
- les substituants de R2 sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R3 représente un radical de formule -C₆H_{(5-y')}-B_{y'} pour lequel y' = 1, 2 ou 3; ou un radical de formule -A-C₆H_{(5-y)}-B_{y} pour lequel y = 0, 1 ou 2; et/ou
- le radical A de R3 est un radical divalent hydrocarboné, linéaire ou ramifié, saturé ou insaturé, ayant 1 à 12 atomes de carbone, éventuellement substitué; et/ou
- le radical B de R3 est un radical hydrocarboné, linéaire ou ramifié, saturé ou insaturé, ayant 1 à 12 atomes de carbone, éventuellement substitué; et/ou
- les substituants de A et/ou de B sont choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné; et/ou
- le radical X représente un radical choisi parmi -OH ou -OR₄ avec R₄ représentant un radical hydrocarboné, linéaire, cyclique ou ramifié, saturé ou insaturé, ayant 1 à 6 atomes de carbone, éventuellement substitué; et/ou
- les substituants de R4 de X sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

3. Composition selon l'une des revendications 1 ou 2, dans laquelle le composé de formule (I) est tel que :
- le radical R1 représente un radical méthyle, éthyle, propyle ou isopropyle, éventuellement substitué par un groupement -OH ou -P(O)-(OR)₂ avec R représentant méthyle, éthyle, propyle ou isopropyle; et/ou
- le radical R2 représente un radical méthyle, éthyle, propyle, isopropyle, n-butyle, ter-butyle ou isobutyle; et/ou
- le radical R3 représente un groupement choisi parmi l'une des formules suivantes : 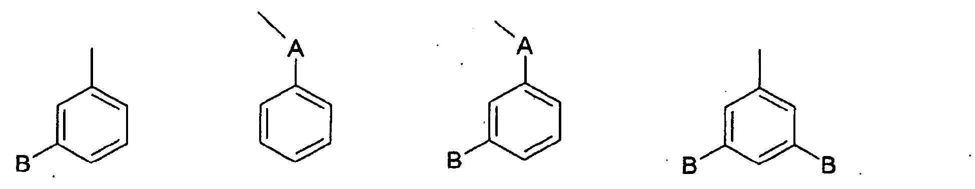 dans lesquelles le radical divalent A est un méthylène, un éthylène, un propylène et/ou le radical B est un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF3).
- le radical X représente un radical choisi parmi -OH, -OCH₃, -OC₂H₅, -O-C₃H₇ ou -OC₄H₉.

4. Composition selon l'une des revendications 1 à 3, dans laquelle le composé de formule (I) est choisi parmi les composés suivants :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

5. Composition selon l'une des revendications précédentes, dans laquelle le composé inhibiteur de l'élastase de la famille des N-Acylaminoamides est présent en une quantité comprise entre 0,00001 et 20% en poids par rapport au poids total de la composition, notamment entre 0,001 et 10% en poids, et de préférence entre 0,05 et 5% en poids.

6. Composition selon l'une des revendications précédentes, **caractérisée en ce que** l'agent myorelaxant est choisi parmi un inhibiteur des canaux calciques et/ou un agoniste des canaux chlore.

7. Composition selon la revendication 6, **caractérisée en ce que** l'inhibiteur de canaux calciques est choisi parmi un inhibiteur agissant sur la membrane plasmique et un inhibiteur agissant de manière intracellulaire.

8. Composition selon la revendication 6 ou 7, **caractérisée en ce que** l'inhibiteur de canaux calciques est l'Alvérine et/ou ses sels organiques ou inorganiques et/ou ses dérivés et/ou ses analogues et/ou le Manganèse et/ou ses sels organiques ou inorganiques.

9. Composition selon l'une des revendications 6 ou 7, **caractérisée en ce que** l'inhibiteur de canaux calciques est choisi parmi phenylalkylamines comme par exemple le vérapamil, l'anipamil, le gallopamil, le dévapamil, le falipamil, le tiapamil,

10. Composition selon l'une des revendications 6 ou 7, **caractérisée en e que** l'inhibiteur de canaux calciques est choisi parmi des dihydropyridines comme par exemple la nifédipine, l'amlodipine, la dazodipine, la félodipine, l'isradipine, lanicardipine, la nimodipine, la nisoldipine, la nitrendipine, la ryosidine.

11. Composition selon l'une des revendications 6 ou 7, **caractérisée en ce que** l'inhibiteur de canaux calciques est choisi parmi des benzothiazépines comme par exemple le Diltiazem, des diphenylpipérazines comme par exemple la cinnarizine, la flunarizine ;

12. Composition selon l'une des revendications 6 ou 7, **caractérisée en ce que** l'inhibiteur de canaux calciques est choisi parmi des agents intervenant au niveau du réticulum sarcoplasmique comme par exemple le dantrolène et le TMB-8.

13. Composition selon l'une des revendications 6 ou 7, **caractérisée en ce que** l'inhibiteur de canaux calciques est choisi parmi des antagonistes de la calmoduline comme par exemple la phénothiazine, la trifluopérazine, la chlorpromazine ou des dérivés du naphtalène

14. Composition selon l'une des revendications 6 ou 7, **caractérisée en ce que** l'inhibiteur de canaux calciques est choisi parmi des anesthésiques locaux comme la dibucaïne ou des antagonistes de la dopamine comme la pimozide, l'halopéridol ou le calmidazolium.

15. Composition selon la revendication 6, **caractérisée en ce que** l'agoniste des canaux chlore est choisi parmi des benzodiazepines, des analogues du GABA, mimétique du récepteur GABA ergique, mais également de la glycine et/ou de ses dérivés.

16. Composition selon l'une des revendications 6 à 15, **caractérisée en ce qu'**elle comprend à la fois au moins un inhibiteur de canaux calciques et au moins un agoniste des canaux chlore.

17. Composition selon l'une des revendications précédentes, se présentant sous la forme d'une composition cosmétique ou dermatologique destinée au soin et/ou au traitement des zones ulcérées ou ayant subi un stress ou microstress cutané, notamment généré par une exposition aux UV et/ou la mise en contact avec un produit irritant.

18. Composition selon l'une des revendications 1 à 15, se présentant sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

19. Composition selon l'une des revendications 1 à 15, se présentant sous la forme d'une composition de soin de la peau du visage, de type anti-rides ou anti-age, ou d'une composition de protection solaire ou après-soleil.

20. Procédé de traitement cosmétique des rides et/ou des ridules, consistant à appliquer par voie topique une composition cosmétique telle que définie dans l'une des revendications 1 à 19.

## Claims

1. Cosmetic or dermatological composition **characterized in that** it comprises a combination of an inhibitor of elastase of the N-acylaminoamide family and at least one myorelaxing agent, the said inhibitory compound of the N-acylaminoamide family is a compound of formula (I): 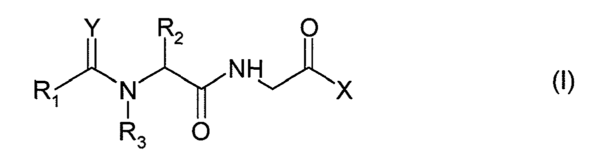 in which:
- the Y radical represents O or S,
- the R1 radical represents:
- (i) a hydrogen atom
- (ii) a hydrocarbon radical, linear, branched or cyclic, saturated or unsaturated, containing from 1 to 18 carbon atoms,
optionally substituted by 1 to 5 groups, identical or different, selected from -OH; -OR; -O-COR; -SH; -SR; -S-COR; -NH₂; -NHR; -NRR'; -NH-COR; -Hal (halogen); -CN; -COOR; -COR; -P(O)-(OR)₂; -SO₂-OR;
with R and R' each representing independently a hydrocarbon radical, linear, branched or cyclic, saturated or unsaturated, containing 1 to 6 carbon atoms, optionally halogenated, even perhalogenated, the said R and R' radicals being capable of forming together with N a 5- or 6-membered carbon ring that may include in addition at least one heteroatom selected from O, N and/or S in the ring, and/or may be substituted by 1 to 5 groups, identical or different, selected from -OH; -OR"; -O-COR"; -SH; -SR"; -S-COR"; -NH₂; -NHR"; -NH-COR"; - Hal (halogen); -CN; -COOR"; -COR"; with R" representing independently a hydrocarbon radical, linear, branched or cyclic, saturated or unsaturated, containing 1 to 6 carbon atoms, optionally halogenated, even perhalogenated;
- (iii) a radical selected from the radicals -OR; -NH₂; -NHR; - NRR'; -NH-COR; -COOR; -COR;
with R and R' each representing independently a hydrocarbon radical, linear, branched or cyclic, saturated or unsaturated, containing 1 to 6 carbon atoms, optionally halogenated, even perhalogenated, the said R and R' radicals being capable of forming together with N a 5- or 6-membered carbon ring that may include in addition at least one heteroatom selected from O, N and/or S in the ring, and/or may be substituted by 1 to 5 groups, identical or different, selected from -OH; -OR"; -O-COR"; -SH; -SR"; -S-COR"; -NH₂; -NHR"; -NH-COR"; - Hal (halogen); -CN; -COOR"; -COR"; with R" representing a hydrocarbon radical, linear, branched or cyclic, saturated or unsaturated, containing 1 to 6 carbon atoms, optionally halogenated, even perhalogenated;
- the radical R2 represents a hydrocarbon radical, linear, branched or
cyclic saturated or unsaturated, containing 1 to 18 carbon atoms, optionally substituted by 1 to 5 groups, identical or different, selected from -OH; -OR; -O-COR; -SH; -SR; -S-COR; -NH₂; -NHR; -NRR'; -NH-COR; -Hal (halogen); -CN; -COOR; -COR;
with R and R' each representing independently a hydrocarbon radical, linear, branched or cyclic, saturated or unsaturated, containing 1 to 6 carbon atoms, optionally halogenated, even perhalogenated, the said R and R' radicals being capable of forming together with N a 5- or 6-membered carbon ring that may include in addition at least one heteroatom selected from O, N and/or S in the ring, and/or may be substituted by 1 to 5 groups, identical or different, selected from -OH; - OR"; -O-COR"; -SH; -SR"; -S-COR"; -NH₂; -NHR"; -NH-COR"; -Hal (halogen); -CN; -COOR"; -COR"; with R" representing a hydrocarbon radical, linear, branched or cyclic, saturated or unsaturated, containing 1 to 6 carbon atoms, optionally halogenated, even perhalogenated;
- the radical R3 represents a radical selected from those of formula (II) or (III)
(II) -A-C₆H_{(5-y)}-B_{y}
(III) -C₆H_{(5-y)}-B_{y'}
in which:
- y is an integer included between 0 and 5, and y' is an integer included between 1 and 5;
- A is a divalent hydrocarbon radical, linear, branched or cyclic, saturated or
unsaturated, containing 1 to 18 carbon atoms,
optionally substituted by 1 to 5 groups, identical or different, selected from -OH; -OR; -O-COR; -SH; -SR; -S-COR; -NH₂; -NHR; -NRR'; -NH-COR;
- Hal (halogen, even perhalogen); -CN; -COOR; -COR; -NO₂; -SO₂-OR;
with R and R' each representing independently a hydrocarbon radical, linear, branched or cyclic, saturated or unsaturated, containing 1 to 6 carbon atoms, optionally halogenated, even perhalogenated, the said R and R' radicals being capable of forming together with N a 5- or 6-membered carbon ring that may include in addition at least one heteroatom selected from O, N and/or S in the ring, and/or may be substituted by 1 to 5 groups, identical or different, selected from -OH; -OR"; -O-COR"; -SH; -SR"; -S-COR"; -NH₂; -NHR"; -NH-COR"; - Hal (halogen); -CN; -COOR"; -COR"; with R" representing a hydrocarbon radical, linear, branched or cyclic, saturated or unsaturated, containing 1 to 6 carbon atoms, optionally halogenated, even perhalogenated;
- B is a hydrocarbon radical, linear, branched or cyclic, saturated
or unsaturated, containing 1 to 18 carbon atoms,
optionally substituted by 1 to 5 groups, identical or different, selected from -OH; -OR; -O-COR; -SH; -SR; -S-COR; -NH₂; -NHR; -NRR'; -NH-COR; -Hal (halogen, even perhalogen); -CN; -COOR; -COR; -NO₂; - SO₂-OR;
with R and R' each representing independently a hydrocarbon radical, linear, branched or cyclic, saturated or unsaturated, containing 1 to 6 carbon atoms, optionally halogenated, even perhalogenated, the said R and R' radicals being capable of forming together with N a 5- or 6-membered carbon ring that may include in addition at least one heteroatom selected from O, N and/or S in the ring, and/or may be substituted by 1 to 5 groups, identical or different, selected from -OH; - OR"; -O-COR"; -SH; -SR"; -S-COR"; -NH₂; -NHR"; -NH-COR"; -Hal (halogen); -CN; -COOR"; -COR"; with R" representing a hydrocarbon radical, linear, branched or cyclic, saturated or unsaturated, containing 1 to 6 carbon atoms, optionally halogenated, even perhalogenated;
- the radical X represents a radical selected from -OH; -OR₄, -NH₂, -
NHR₄, NR₄R₅, -SR₄, -COOR₄; -COR₄;
with R₄ and R₅ each representing independently a hydrocarbon radical, linear, branched or cyclic, saturated or unsaturated, containing 1 to 6 carbon atoms,
optionally substituted by 1 to 5 groups, identical or different, selected from -OH; -OR; -O-COR; -SH; -SR; -S-COR; -NH₂; -NHR; -NRR'; -NH-COR; -Hal (halogen, even perhalogen); -CN; -COOR; -COR;
with R and R' each representing independently a hydrocarbon radical, linear, branched or cyclic, saturated or unsaturated, containing 1 to 6 carbon atoms, optionally halogenated, even perhalogenated, the said R and R' radicals being capable of forming together with N a 5- or 6-membered carbon ring that may include in addition at least one heteroatom selected from O, N and/or S in the ring, and/or may be substituted by 1 to 5 groups, identical or different, selected from -OH; - OR"; -O-COR"; -SH; -SR"; -S-COR"; -NH₂; -NHR"; -NH-COR"; -Hal (halogen); -CN; -COOR"; -COR"; with R" representing a hydrocarbon radical, linear, branched or cyclic, saturated or unsaturated, containing 1 to 6 carbon atoms, optionally halogenated, even perhalogenated;
the said R₄ and R₅ radicals being capable of forming together with N a 5- or 6- membered carbon ring that may include in addition at least one heteroatom selected from O, N and/or S in the ring, and/or may be substituted by 1 to 5 groups, identical or different, selected from -OH; - OR"; -O-COR"; -SH; -SR"; -S-COR"; -NH₂; -NHR"; -NH-COR"; -Hal (halogen); -CN; -COOR"; -COR"; with R" representing a hydrocarbon radical, linear, branched or cyclic, saturated or unsaturated, containing 1 to 6 carbon atoms, optionally halogenated" even perhalogenated;
its mineral or organic acid salts, its optical isomers, in isolated form or as a racemic mixture.

2. Composition according to claim 1 in which the compound of formula (I) is such that:
- the radical Y represents oxygen, and/or
- the radical R1 represents hydrogen or a hydrocarbon radical, linear or branched, saturated or unsaturated, containing 1 to 12, and in particular 1, 2, 3, 4, 5 or 6 carbon atoms, optionally substituted, and/or
- the substituents of R1 are selected from -OH, -OR and/or - P(O)-(OR)₂ with R representing a hydrocarbon radical, linear, branched or cyclic, saturated or unsaturated, containing 1 to 6 carbon atoms, optionally halogenated, even perhalogenated; and/or
- the radical R2 represents a hydrocarbon radical, linear, branched or cyclic, saturated or unsaturated, containing 1 to 12, and in particular 1, 2, 3, 4, 5 or 6 carbon atoms, optionally substituted; and/or
- the substituents of R2 are selected from -OH and -OR with R representing a hydrocarbon radical, linear, branched or cyclic, saturated or unsaturated, containing 1 to 6 carbon atoms, optionally halogenated, even perhalogenated; and/or
- the radical R3 represents a radical of formula -C₆H_{(5-y')} -B_{y'} for which y'= 1, 2 or 3; or a radical of formula -A-C₆H_{(5-y)} -B_{y} for which y = 0, 1 or 2; and/or
- the radical A of R3 is a divalent hydrocarbon radical, linear or branched, saturated or unsaturated, containing 1 to 12 carbon atoms, optionally substituted; and/or
- the radical B of R3 is a hydrocarbon radical, linear or branched, saturated or unsaturated, containing 1 to 12 carbon atoms, optionally substituted; and/or
- the substituents of A and/or B are selected from -Hal (halogen, even perhalogen); -CN; -COOR; -NO₂; -SO₂-OR; with R representing a hydrocarbon radical, linear, branched or cyclic, saturated or unsaturated, containing 1 to 6 carbon atoms, optionally halogenated, even perhalogenated; and/or
- the radical X represents a radical selected from -OH or OR₄ with R₄ representing a hydrocarbon radical, linear, cyclic or branched, saturated or unsaturated, containing 1 to 6 carbon atoms, optionally substituted;
- the substituents of R4 of X are chosen from -OH and -OR with R representing a hydrocarbon radical, linear, branched or cyclic, saturated or unsaturated, containing 1 to 6 carbon atoms, optionally halogenated, even perhalogenated.

3. Composition according to one of the claims 1 or 2, in which the compound of
formula (I) is such that:
- the radical R1 represents a methyl, ethyl, propyl or isopropyl radical, optionally substituted by an -OH or -P(O)-(OR)₂ group with R representing methyl, ethyl, propyl or isopropyl; and/or
- the radical R2 represents a methyl, ethyl, propyl, isopropyl, n-butyl, tert-butyl or isobutyl radical; and/or
- the radical R3 represents a group selected from one of the following formulae: 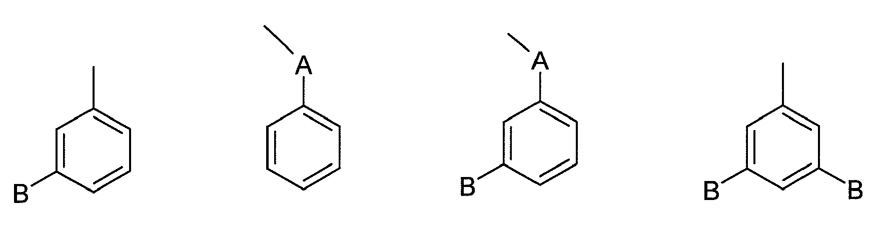
in which the divalent radical A is methylene, ethylene, propylene and/or the radical B is a methyl, ethyl, propyl or isopropyl radical substituted by one or more halogens, in particular chlorine, bromine, iodine or fluorine, and preferably completely halogenated (perhalogenated), such as perfluorinated, in particular the perfluoromethyl radical (-CF3).
- the radical X represents a radical selected from -OH, -OCH₃, - OC₂H₅, -OC₃H₇ or -OC₄H₉.

4. Composition according to one of the claims 1 to 3, in which the compound of formula (I) is selected from the following compounds:
- {2-[acetyl-(3-trifluoromethyl-phenyl)-amino]-3-methyl-butyrylamino} acetic acid
- {2-[acetyl-(3-trifluoromethyl-phenyl)-amino]-3-methyl-butyrylamino} ethyl acetate
- [2-(acetyl-benzyl-amino)-3-methyl-butyrylamino] acetic acid
- [2-(acetyl-benzyl-amino)-3-methyl-butyrylamino] ethyl acetate
- (2-{benzyl-[(diethoxy-phosphoryl)-acetyl]-amino}-3-methyl-butyrylamino) ethyl acetate.

5. Composition according to one of the preceding claims, in which the inhibitor of elastase of the N-acylaminoamide family is present in a quantity included between 0.00001 and 20% by weight of the total weight of the composition, in particular between 0.001 and 10% by weight and preferably between 0.05 and 5% by weight.

6. Composition according to one of the preceding claims, **characterized in that** the myorelaxing agent is selected from a calcium channel inhibitor and/or a chloride channel agonist.

7. Composition according to claim 6, **characterized in that** the calcium channel inhibitor is selected from an inhibitor acting on the plasma membrane and an inhibitor acting intracellularly.

8. Composition according to claim 6 or 7, **characterized in that** the calcium channel inhibitor is Alverine and/or its organic or inorganic salts and/or its derivatives and/or its analogues and/or manganese and/or its organic or inorganic salts.

9. Composition according to one of the claims 6 or 7, **characterized in that** the calcium channel inhibitor is selected from phenylalkylamines like for example verapamil, anipamil, gallopamil, devapamil, falipamil, tiapamil.

10. Composition according to one of the claims 6 or 7, **characterized in that** the calcium channel inhibitor is selected from dihydropyridines like for example nifedipine, amlodipine,dazodipine, felodipine, isradipine, lanicardipine, nimodipine, nisoldipine, nitrendipine, ryosidine.

11. Composition according to one of the claims 6 or 7, **characterized in that** the calcium channel inhibitor is selected from benzothiazepines like for example diltiazem, diphenylpiperazines like for example cinnarizine, flunarizine;

12. Composition according to one of the claims 6 or 7, **characterized in that** the calcium channel inhibitor is selected from agents intervening at the level of the sarcoplasmic reticulum like for example dantrolene and TMB-8.

13. Composition according to one of the Claims 6 or 7, **characterized in that** the calcium channel inhibitor is selected from calmodulin antagonists like for example phenothiazine, trifluoperazine, chlorpromazine or naphthalene derivatives.

14. Composition according to one of the claims 6 or 7, **characterized in that** the calcium channel inhibitor is selected from local anesthetics like dibucaine or dopamine antagonists like pimozide, haloperidol or calmidazolium.

15. Composition according to claim 6, **characterized in that** the chloride channel agonist is selected from benzodiazepine, analogues of GABA, mimetic of the GABAergic receptor, but also glycine and/or its derivatives.

16. Composition according to one of the claims 6 to 15, **characterized in that** it contains both at least one calcium channel inhibitor and at least one chlorine channel agonist.

17. Composition according to one of the preceding Claims presented in the form of a cosmetic or dermatological composition designed for the care and/or treatment of ulcerated areas or areas subjected to a cutaneous stress or microstress, in particular generated by exposure to UV and/or being placed in contact with an irritant product.

18. Composition according to one of the Claims 1 to 15, presented in the form of:
- a product for the care, treatment, cleansing or protection of the skin of the face or body including the scalp, such as a care composition (daytime, nighttime, hydrating) for the face or body; an anti-wrinkle or anti-age composition for the face; a composition rendering the face mat; a composition for irritated skin;
- a sun protection composition, an artificial tanning composition (self-tanning) or care composition after exposure to the sun;
- a composition for the hair, and in particular a sun protection cream or gel; a care composition for the scalp, in particular against hair loss or stimulating hair growth;
- a product for the make-up of the skin or the face, body or lips, such as foundation make-up, complexion cream, blusher or eye-shadow, a free or compact powder, anti-shadow stick, a camouflaging stick, a lipstick, a lip care composition;
- a product for buccal hygiene such as toothpaste or a mouthwash.

19. Composition according to one of the claims 1 to 15, presented in the form of a care composition for the skin of the face of the anti-wrinkle or anti-age type, or of a sun protection or après-soleil composition.

20. Cosmetic treatment method of wrinkles and/or skin creases, consisting of the topical application of a cosmetic composition such as defined in one of the claims 1 to 19.

## Patentansprüche

1. Kosmetische oder dermatologische Zusammensetzung, **dadurch gekennzeichnet, dass** sie eine Kombination aus einem Elastase-Inhibitor aus der Familie der N-Acylaminoamide und wenigstens ein Muskelrelaxans umfasst, wobei der Inhibitor aus der Familie der N-Acylaminoamide Folgendes ist: eine Verbindung der Formel (I): 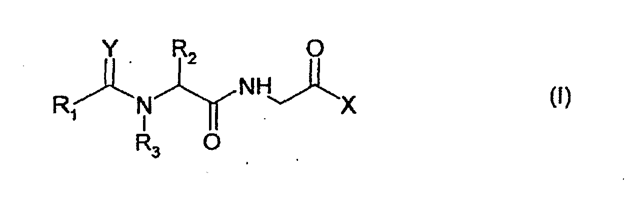 wobei:
- der Rest Y für O oder S steht;
- der Rest R₁ für Folgendes steht:
- (i) ein Wasserstoffatom;
- (ii) einen linearen, verzweigten oder cyclischen, gesättigten oder ungesättigten Kohlenwasserstoffrest mit 1 bis 18 Kohlenstoffatomen, der gegebenenfalls mit 1 bis 5 gleichen oder verschiedenen Gruppen substituiert ist, die aus -OH, -OR, -O-COR, -SH, -SR, -S-COR, -NH₂, -NHR, -NRR', -NH-COR, -Hal (Halogen), -CN, -COOR, -COR, -P(O)(OR)₂, -SO₂-OR ausgewählt sind;
wobei R und R' unabhängig voneinander für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, stehen;
wobei die Reste R und R' zusammen mit N einen Kohlenstoffring mit 5 oder 6 Ringgliedern bilden können, die weiterhin wenigstens ein Heteroatom, das aus O, N und/oder S ausgewählt ist, im Ring umfassen können und/oder mit 1 bis 5 gleichen oder verschiedenen Gruppen substituiert sein können, die aus -OH, -OR", -O-COR", -SH, -SR", -S-COR", -NH₂, -NHR", -NH-COR", -Hal (Halogen), -CN, -COOR", -COR" ausgewählt sind, wobei R" für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht;
- (iii) einen Rest, der aus den Resten -OR, -NH₂, -NHR, -NRR', -NH-COR, -COOR, -COR ausgewählt ist;
wobei R und R' unabhängig voneinander für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, stehen;
wobei die Reste R und R' zusammen mit N einen Kohlenstoffring mit 5 oder 6 Ringgliedern bilden können, die weiterhin wenigstens ein Heteroatom, das aus O, N und/oder S ausgewählt ist, im Ring umfassen können und/oder mit 1 bis 5 gleichen oder verschiedenen Gruppen substituiert sein können, die aus -OH, -OR", -O-COR", -SH, -SR", -S-COR", -NH₂, -NHR", -NH-COR", -Hal (Halogen), -CN, -COOR", -COR" ausgewählt sind, wobei R" für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht;
- der Rest R₂ für einen linearen, verzweigten oder cyclischen, gesättigten oder ungesättigten Kohlenwasserstoffrest mit 1 bis 18 Kohlenstoffatomen, der gegebenenfalls mit 1 bis 5 gleichen oder verschiedenen Gruppen substituiert ist, die aus -OH, -OR, -O-COR, -SH, -SR, -S-COR, -NH₂, -NHR, -NRR', -NH-COR, -Hal (Halogen), -CN, -COOR, -COR ausgewählt sind;
wobei R und R' unabhängig voneinander für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, stehen;
wobei die Reste R und R' zusammen mit N einen Kohlenstoffring mit 5 oder 6 Ringgliedern bilden können, die weiterhin wenigstens ein Heteroatom, das aus O, N und/oder S ausgewählt ist, im Ring umfassen können und/oder mit 1 bis 5 gleichen oder verschiedenen Gruppen substituiert sein können, die aus -OH, -OR", -O-COR", -SH, -SR", -S-COR", -NH₂, -NHR", -NH-COR", -Hal (Halogen), -CN, -COOR", -COR" ausgewählt sind, wobei R" für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht;
- der Rest R₃ für einen Rest steht, der aus solchen der Formel (II) oder (III) ausgewählt ist:
(II) -A-C₆H_{(5-y)}-B_{y}
(III) -C₆H_{(5-y')}-B_{y'}
wobei:
- y eine ganze Zahl von 0 bis 5 ist und y' eine ganze Zahl von 0 bis 5 ist;
- A ein zweiwertiger linearer oder verzweigter, gesättigter oder ungesättigter Kohlenwasserstoffrest mit 1 bis 18 Kohlenstoffatomen ist, der gegebenenfalls mit 1 bis 5 gleichen oder verschiedenen Gruppen substituiert ist, die aus -OH, -OR, -O-COR, -SH, -SR, -S-COR, -NH₂, -NHR, -NRR', -NH-COR, -Hal (halogeniert oder sogar perhalogeniert), -CN, -COOR, -COR, -NO₂, -SO₂-OR ausgewählt sind;
wobei R und R' unabhängig voneinander für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, stehen;
wobei die Reste R und R' zusammen mit N einen Kohlenstoffring mit 5 oder 6 Ringgliedern bilden können, die weiterhin wenigstens ein Heteroatom, das aus O, N und/oder S ausgewählt ist, im Ring umfassen können und/oder mit 1 bis 5 gleichen oder verschiedenen Gruppen substituiert sein können, die aus -OH, -OR", -O-COR", -SH, -SR", -S-COR", -NH₂, -NHR", -NH-COR", -Hal (Halogen), -CN, -COOR", -COR" ausgewählt sind, wobei R" für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht;
- B ein linearer oder verzweigter, gesättigter oder ungesättigter Kohlenwasserstoffrest mit 1 bis 18 Kohlenstoffatomen ist, der gegebenenfalls mit 1 bis 5 gleichen oder verschiedenen Gruppen substituiert ist, die aus -OH, -OR, -O-COR, -SH, -SR, -S-COR, -NH₂, -NHR, -NRR', -NH-COR, -Hal (halogeniert oder sogar perhalogeniert), -CN, -COOR, -COR, -NO₂, -SO₂-OR ausgewählt sind;
wobei R und R' unabhängig voneinander für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, stehen;
wobei die Reste R und R' zusammen mit N einen Kohlenstoffring mit 5 oder 6 Ringgliedern bilden können, die weiterhin wenigstens ein Heteroatom, das aus O, N und/oder S ausgewählt ist, im Ring umfassen können und/oder mit 1 bis 5 gleichen oder verschiedenen Gruppen substituiert sein können, die aus -OH, -OR", -O-COR", -SH, -SR", -S-COR", -NH₂, -NHR", -NH-COR", -Hal (Halogen), -CN, -COOR", -COR" ausgewählt sind, wobei R" für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht;
- der Rest X für einen Rest steht, der aus -OH, -OR₄, -NH₂, -NHR₄, -NR₄R₅, -SR₄, -COOR₄, -COR₄ ausgewählt ist;
wobei R₄ und R₅ unabhängig voneinander für einen linearen, cyclischen oder verzweigten, gesättigten oder ungesättigten Kohlenwasserstoffrest mit 1 bis 6 Kohlenstoffatomen stehen, der gegebenenfalls mit 1 bis 5 gleichen oder verschiedenen Gruppen substituiert ist, die aus -OH, -OR, -O-COR, -SH, -SR, -S-COR, -NH₂, -NHR, -NRR', -NH-COR, -Hal (halogeniert oder sogar perhalogeniert), -CN, -COOR, -COR ausgewählt sind, wobei R und R' unabhängig voneinander für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, stehen, wobei die Reste R und R' zusammen mit N einen Kohlenstoffring mit 5 oder 6 Ringgliedern bilden können, die weiterhin wenigstens ein Heteroatom, das aus O, N und/oder S ausgewählt ist, im Ring umfassen können und/oder mit 1 bis 5 gleichen oder verschiedenen Gruppen substituiert sein können, die aus -OH, -OR", -O-COR", -SH, -SR", -S-COR", -NH₂, -NHR", -NH-COR", -Hal (Halogen), -CN, -COOR", -COR" ausgewählt sind, wobei R" für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht;
wobei die Reste R₄ und R₅ zusammen mit N einen Kohlenstoffring mit 5 oder 6 Ringgliedern bilden können, die weiterhin wenigstens ein Heteroatom, das aus O, N und/oder S ausgewählt ist, im Ring umfassen können und/oder mit 1 bis 5 gleichen oder verschiedenen Gruppen substituiert sein können, die aus -OH, -OR", -O-COR", -SH, -SR", -S-COR", -NH₂, -NHR", -NH-COR", -Hal (Halogen), -CN, -COOR", -COR" ausgewählt sind, wobei R" für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht;
deren Salze einer Mineral- oder organischen Säure, deren optische Isomere in isolierter Form oder als racemisches Gemisch.

2. Zusammensetzung gemäß Anspruch 1, wobei bei der Verbindung der Formel (I):
- der Rest Y für Sauerstoff steht; und/oder
- der Rest R₁ für Wasserstoff oder einen linearen oder verzweigten, gesättigten oder ungesättigten Kohlenwasserstoffrest mit 1 bis 12 und insbesondere 1, 2, 3, 4, 5 oder 6 Kohlenstoffatomen, der gegebenenfalls substituiert ist, steht; und/oder
- die Substituenten von R₁ aus -OH, -OR und/oder -P(O)(OR)₂ ausgewählt sind, wobei R für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht; und/oder
- der Rest R₂ für einen linearen, verzweigten oder cyclischen, gesättigten oder ungesättigten Kohlenwasserstoffrest mit 1 bis 12 und insbesondere 1, 2, 3, 4, 5 oder 6 Kohlenstoffatomen, der gegebenenfalls substituiert ist, steht; und/oder
- die Substituenten von R₂ aus -OH und -OR ausgewählt sind, wobei R für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht; und/oder
- der Rest R₃ für einen Rest der Formel -C₆H_{(5-y')}-B_{y'}, wobei y' = 1, 2 oder 3 ist, oder einen Rest der Formel -A-C₆H_{(5-y)}-B_{y}, wobei y = 0, 1 oder 2 ist, steht; und/oder
der Rest A von R₃ ein zweiwertiger linearer oder verzweigter, gesättigter oder ungesättigter Kohlenwasserstoffrest mit 1 bis 12 Kohlenstoffatomen ist, der gegebenenfalls substituiert ist; und/oder
der Rest B von R₃ ein linearer oder verzweigter, gesättigter oder ungesättigter Kohlenwasserstoffrest mit 1 bis 12 Kohlenstoffatomen ist, der gegebenenfalls substituiert ist; und/oder
- die Substituenten von A und/oder B aus -Hal (halogeniert oder sogar perhalogeniert), -CN, -COOR, -NO₂, -SO₂-OR ausgewählt sind, wobei R für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht; und/oder
- der Rest X für einen Rest steht, der aus -OH oder -OR₄ ausgewählt ist, wobei R₄ für einen linearen, cyclischen oder verzweigten, gesättigten oder ungesättigten Kohlenwasserstoffrest mit 1 bis 6 Kohlenstoffatomen, der gegebenenfalls substituiert ist, steht; und/oder
- die Substituenten von R₄ von X aus -OH und -OR ausgewählt sind, wobei R für einen linearen, verzweigten oder cyclischen, gesättigten oder ungesättigten Kohlenwasserstoffrest mit 1 bis 6 Kohlenstoffatomen, der gegebenenfalls halogeniert oder sogar perhalogeniert ist, steht.

3. Zusammensetzung gemäß einem der Ansprüche 1 oder 2, wobei bei der Verbindung der Formel (I):
- der Rest R₁ für einen Methyl-, Ethyl-, Propyl- oder Isopropylrest steht, der gegebenenfalls mit einer Gruppe -OH oder -P(O)(OR)₂ substituiert ist, wobei R für Methyl, Ethyl, Propyl oder Isopropyl steht; und/oder
- der Rest R₂ für einen Methyl-, Ethyl-, Propyl-, Isopropyl-, n-Butyl-, tert-Butyl- oder Isobutylrest steht; und/oder
- der Rest R₃ für eine Gruppe steht, die aus einer der folgenden Formeln ausgewählt ist: 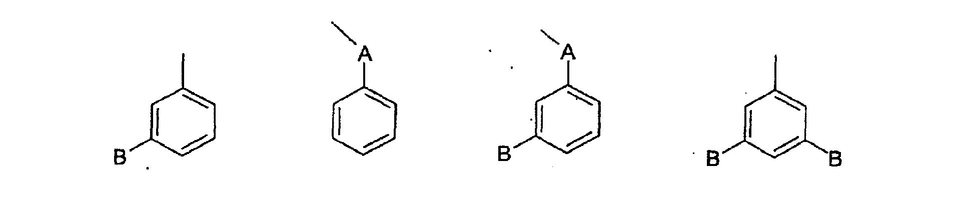 wobei der zweiwertige Rest A ein Methylen, Ethylen, Propylen ist und/oder der Rest B ein Methyl-, Ethyl-, Propyl- oder Isopropylrest ist, der mit einem oder mehreren Halogenen, insbesondere Chlor, Brom, Iod oder Fluor, substituiert und vorzugsweise vollständig halogeniert (perhalogeniert), wie perfluoriert, ist und insbesondere der Perfluormethylrest (-CF₃) ist;
- der Rest X für einen Rest steht, der aus -OH, -OCH₃, -OC₂H₅, -O-C₃H₇ oder -OC₄H₉ ausgewählt ist.

4. Zusammensetzung gemäß einem der Ansprüche 1 bis 3, wobei die Verbindung der Formel (I) aus den folgenden Verbindungen ausgewählt ist:
- {2-[Acetyl(3-trifluormethylphenyl)amino]-3-methylbutyrylamino}essigsäure;
- Ethyl{2-[Acetyl(3-trifluormethylphenyl)amino]-3-methylbutyrylamino}-acetat;
- [2-(Acetylbenzylamino)-3-methylbutyrylamino]essigsäure;
- Ethyl[2-(acetylbenzylamino)-3-methylbutyrylamino] acetat;
- Ethyl(2-{benzyl[(diethoxyphosphoryl)acetyl]amino}-3-methylbutyryl-amino)acetat.

5. Zusammensetzung gemäß einem der vorstehenden Ansprüche, wobei der Elastase-Inhibitor aus der Familie der N-Acylaminoamide in einer Menge zwischen 0,00001 und 20 Gew.-% vorhanden ist, bezogen auf das Gesamtgewicht der Zusammensetzung, insbesondere zwischen 0,001 und 10 Gew.-% und vorzugsweise zwischen 0,05 und 5 Gew.-%.

6. Zusammensetzung gemäß einem der vorstehenden Ansprüche, **dadurch gekennzeichnet, dass** das Muskelrelaxans aus einem Calciumkanal-Inhibitor und/oder einem Chloridkanal-Agonisten ausgewählt ist.

7. Zusammensetzung gemäß Anspruch 6, **dadurch gekennzeichnet, dass** der Calciumkanal-Inhibitor aus einem Inhibitor, der auf die Plasmamembran wirkt, und einem Inhibitor, der intrazellulär wirkt, ausgewählt ist.

8. Zusammensetzung gemäß Anspruch 6 oder 7, **dadurch gekennzeichnet, dass** es sich bei dem Calciumkanal-Inhibitor um Alverin und/oder dessen organische oder anorganische Salze und/oder dessen Derivate und/oder
dessen Analoga und/oder um Mangan und/oder dessen organische oder anorganische Salze handelt.

9. Zusammensetzung gemäß einem der Ansprüche 6 oder 7, **dadurch gekennzeichnet, dass** der Calciumkanal-Inhibitor aus Phenylalkylaminen, wie zum Beispiel Verapamil, Anipamil, Gallopamil, Devapamil, Falipamil, Tiapamil, ausgewählt ist.

10. Zusammensetzung gemäß einem der Ansprüche 6 oder 7, **dadurch gekennzeichnet, dass** der Calciumkanal-Inhibitor aus Dihydropyridinen, wie zum Beispiel Nifedipin, Amlodipin, Dazodipin, Felodipin, Isradipin, Lanicardipin, Nimodipin, Nisoldipin, Nitrendipin, Ryosidin, ausgewählt ist.

11. Zusammensetzung gemäß einem der Ansprüche 6 oder 7, **dadurch gekennzeichnet, dass** der Calciumkanal-Inhibitor aus Benzothiazepinen, wie zum Beispiel Diltiazem, Diphenylpiperazinen, wie zum Beispiel Cinnarizin, Flunarizin, ausgewählt ist.

12. Zusammensetzung gemäß einem der Ansprüche 6 oder 7, **dadurch gekennzeichnet, dass** der Calciumkanal-Inhibitor aus Mitteln, die auf der Ebene des sarkoplasmatischen Retikulums eingreifen, wie zum Beispiel Dantrolen und TMB-8, ausgewählt ist.

13. Zusammensetzung gemäß einem der Ansprüche 6 oder 7, **dadurch gekennzeichnet, dass** der Calciumkanal-Inhibitor aus Calmodulin-Antagonisten, wie zum Beispiel Phenothiazin, Trifluoperazin, Chlorpromazin oder Naphthalinderivaten, ausgewählt ist.

14. Zusammensetzung gemäß einem der Ansprüche 6 oder 7, **dadurch gekennzeichnet, dass** der Calciumkanal-Inhibitor aus Lokalanästhetika, wie Dibucain, oder Dopamin-Antagonisten, wie Pimozid, Haloperidol oder Calmidazolium, ausgewählt ist.

15. Zusammensetzung gemäß Anspruch 6, **dadurch gekennzeichnet, dass** der Chloridkanal-Agonist aus Benzodiazepinen, GABA-Analoga, Mimetika des GABA-Rezeptors, aber auch Glycin und/oder dessen Derivaten, ausgewählt ist.

16. Zusammensetzung gemäß einem der Ansprüche 6 bis 15, **dadurch gekennzeichnet, dass** sie gleichzeitig wenigstens einen Calciumkanal-Inhibitor und wenigstens einen Chloridkanal-Agonisten umfasst.

17. Zusammensetzung gemäß einem der vorstehenden Ansprüche, die in Form einer kosmetischen oder dermatologischen Zusammensetzung für die Pflege und/oder Behandlung von ulzerierten Zonen oder von Zonen, die einen kutanen Stress oder Mikrostress erlitten haben, der insbesondere durch eine Einwirkung von UV-Strahlen und/oder Kontakt mit einem reizenden Produkt erzeugt wurde, vorliegt.

18. Zusammensetzung gemäß einem der Ansprüche 1 bis 15, die vorliegt als:
- Produkt zur Pflege, Behandlung, Reinigung oder zum Schutz der Haut des Gesichts oder des Körpers einschließlich der Kopfhaut, wie als Zusammensetzung zur Pflege (Tag-, Nacht-, Feuchtigkeits-) des Gesichts oder des Körpers; Anti-Falten- oder Anti-Ageing-Zusammensetzung für das Gesicht; mattierende Zusammensetzung für das Gesicht; Zusammensetzung für gereizte Haut;
- Zusammensetzung zum Sonnenschutz, zur künstlichen Bräunung (Selbstbräuner) oder zur After-Sun-Pflege;
- Haarzusammensetzung und insbesondere Sonnenschutzcreme oder -gel; Kopfhautpflegezusammensetzung, insbesondere gegen Haarausfall oder zur Stimulierung des Haarwachstums;
- Produkt zum Schminken der Haut des Gesichts, des Körpers oder der Lippen, wie eine Foundation, eine getönte Tagescreme, Rouge oder Lidschatten, freies oder kompaktes Pulver, Stift gegen Augenringe, Abdeckstift, Lippenstift, Lippenpflege;
- Mundhygieneprodukt, wie Zahnpasta oder Mundspülung.

19. Zusammensetzung gemäß einem der Ansprüche 1 bis 15, die in Form einer Zusammensetzung zur Pflege der Gesichtshaut des Antifalten oder Anti-Ageing-Typs oder einer Zusammensetzung zum Sonnenschutz oder zur After-Sun-Pflege vorliegt.

20. Verfahren zur kosmetischen Behandlung von Falten und/oder Fältchen, das darin besteht, eine Kosmetikzusammensetzung, wie sie in einem der Ansprüche 1 bis 19 definiert ist, topisch zu applizieren.
